# EUROPEAN PATENT APPLICATION

(11) **EP 1 366 733 A2**
(43) Date of publication of application: **03.12.2003**
(21) Application number: 03253292.1
(22) Date of filing: 27.05.2003
(51) Int. Cl.: A61F 2/44

(54) **Spinal prosthesis**

(30) Priority: 27.05.2002 GB 0212150
(71) Applicant: Biomet Merck Limited, Bridgend, South Wales CF31 3XA (GB)
(72) Inventor: Gibson, Alistair, Edinburgh EH10 7JQ (GB); Truscott, James William, Highworth, Swindon SN6 7PG (GB)
(74) Representative: Giles, Ashley Simon

(57) **Abstract**

A vertebral prosthesis comprises a distal element 2 adapted to be received in a distal vertebra, a proximal element 5 adapted to be received in a proximal vertebra, the distal element 2 being capable of articulation relative to the proximal element 5. Preferably a bearing element 4 is attached to the distal element 2 and includes a convex bearing surface 32 which, in vivo, contacts a concave bearing surface 42 of the proximal element 5.

## Description

This invention relates to a spinal prosthesis, and particularly, although not exclusively, relates to a spinal prosthesis which is used to replace a vertebral body, such as a cervical disc.

### BACKGROUND TO THE INVENTION

The cervical spine is a complex anatomic structure, consisting of seven vertebrae supported by ligaments, muscles and intervertebral discs. There are two atypical vertebrae, C1 and C2, which differ in structure and function from all the other vertebrae of the spine. The five typical vertebrae have features similar in form to all the other vertebrae of the spine. That is, they have an anterior body, articular and spinous processes, an intervertebral foramen, and superior and inferior facet joints. The anterior bodies also have lateral buttresses, the unciform processes, which largely restrict motion to flexion and extension. These vertebrae also have transverse foramen which are pathways for the vertebral arteries.

The relative positions of the vertebrae are maintained principally through the anterior and posterior longitudinal ligaments, which lie anterior and posterior to the vertebral body, and the facet joints. The spinous processes are linked by the interspinous ligaments, and the transverse processes are linked by the intertransverse ligaments. The facet joints, true synovial joints, are inclined at approximately 45 degrees. They serve to guide motion between the levels, but do not constrain it.

The intervertebral discs are interposed between the endplates of adjacent vertebrae. Their principal functions are to withstand compression loads and restrain excessive motion. They are comprised of two major elements, the nucleus pulposus and annulus fibrosis. The nucleus pulposus is a gelatinous mass at the core of the disc and functions as a load bearing shock absorber. The annulus fibrosus is a fibrous outer ring surrounding the nucleus. Fibers of the annulus are woven into the outer ring of the adjacent body endplates, and withstand shear and torsional loading.

The cervical portion of the vertebral column possesses the highest degree of mobility, and is the most susceptible to functional stress, trauma, and degenerative arthritic diseases. Symptoms of cervical disc degeneration include nerve root entrapment concomitant with loss of function, pain, and possible disability. Vertebral artery insufficiency may result from osteophytes impinging at the intervertebral foramen.

Treatment of these conditions involves surgical removal of diseased bony material and fusion of the anterior bodies at the levels involved. This enforced partial column rigidity often induces high loading concentrations at adjacent levels, and accelerated decrepitude. Furthermore, the range of movement in the treated area is greatly reduced.

### SUMMARY OF THE INVENTION

According to the present invention there is provided a prosthesis for replacing a part of a human or animal spine, the prosthesis comprising a first element which is adapted to be connected to a first vertebra and a second element which is adapted to be connected to a second vertebra, the first element being capable of articulation relative to the second element in vivo.

Although the first element may articulate directly with the second element, preferably the prosthesis further comprises a bearing element which is interposed between the first and second elements in vivo. The bearing element may be attached to the first element, and the second element may have a bearing surface which engages a cooperating wear surface of the bearing element. The wear surface of the bearing element and the bearing surface of the second element are preferably shaped to reduce the likelihood of dislocation. For example, the wear surface of the bearing element may be convex and the bearing surface of the second element may be concave. Alternatively, the wear surface of the bearing element may be concave and the bearing surface of the second element convex.

The first and second elements are preferably made from stainless steel or titanium. The bearing element is preferably made from ultra high molecular weight polyethylene. Cooperating formations may be provided on the bearing element to engage cooperating formations on the first element.

The bearing element may be fixed permanently to the first element. For example, it may be compression moulded onto the first element.

In accordance with the present invention, a plurality of bearing elements of different dimensions (for example different thickness and/or offset) may be provided; each bearing element may be fixed to a different first element or may be selectively engagable with a common first element.

Preferably, the first element and the second element are cast of a bio-compatible high strength material such as steel or titanium, or an alloy such as a cobalt-chromium-molybdenum alloy or other suitable material.

Preferably, the first and/or second elements are provided with raised projections which are adapted to be secured in bone. Preferably, these projections may be adapted for cemented fixation within the vertebrae. For example, they may be provided with an hydroxyapatite coating to facilitate bone growth in this region.

### BRIEF DESCRIPTION OF THE DRAWINGS

For a better understanding of the present invention and to show how it may be carried into effect, reference will now be made by way of example to the accompanying drawings, in which:-
Figure 1 is a side view of a distal element and a bearing element of a vertebral prosthesis;
Figure 2 is a top view of the distal element of Figure 1;
Figure 3 is a cross-section through the distal element and bearing element of Figure 1 viewed on the line 3-3;
Figure 4 is a side view of the distal element of Figure 1 without the bearing element;
Figure 5 is an underside view of the distal element of Figure 4;
Figure 6 is a side view of a proximal element of the vertebral prosthesis;
Figure 7 is a cross-section through the proximal element of Figure 6 viewed from the line 7-7;
Figure 8 is an underside view of the proximal element of Figure 6.

### DETAILED DESCRIPTION

The following references to the "proximal" element relate to the element closest to the head of the patient, in vivo, and references to the "distal" element refer to the element furthest from the head of the patient, in vivo.

Referring to the drawings, a vertebral prosthesis 1 in accordance with the present invention comprises a distal element 2 which is attached to a bearing element 4 and articulates with a proximal element 5.

The distal element 2 comprises an oval shaped tray 8 which is integrally formed with a vertebral, stem 6. The vertebral stem 6 extends from the centre of a distal surface 10 of the tray 8 in a direction substantially perpendicular to the distal surface 10 of the tray.

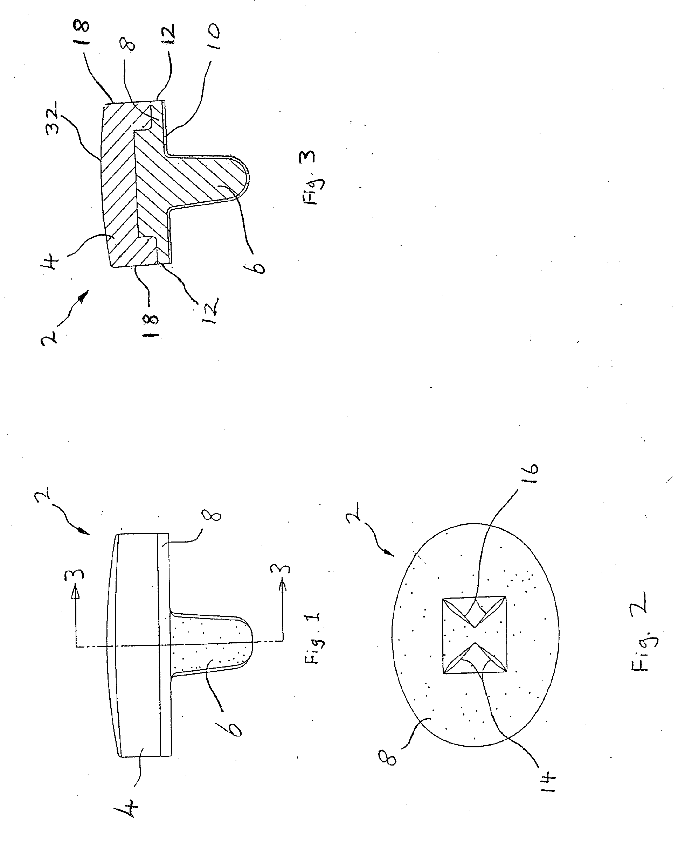

With reference to Figure 3, the distal element 2 has a side wall 12 which defines the periphery of the tray 8. The bearing element 4 is secured to the proximal end of the distal element 2 and has a side wall 18, which is aligned with the side wall 12 of the distal element 2. The proximal face of the bearing element 4 comprises a convex bearing surface 32.

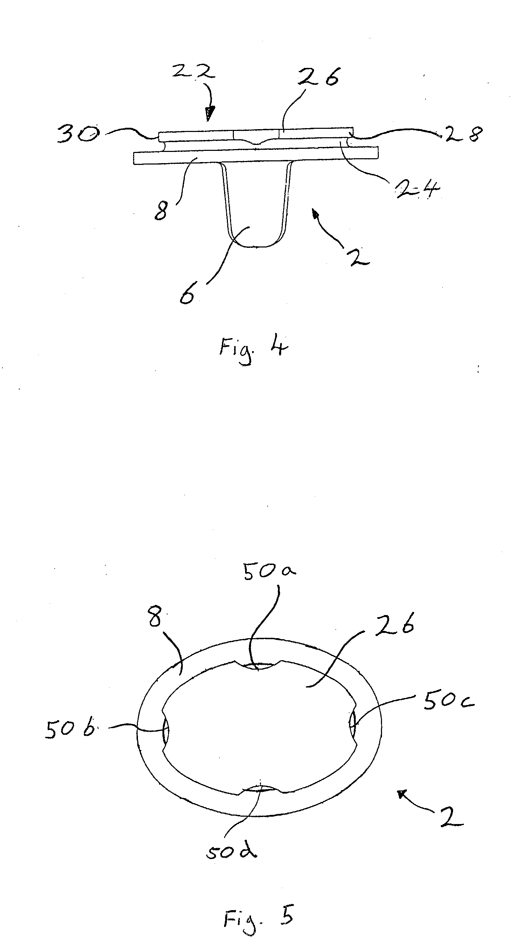

With reference to Figure 4, on the proximal face of the tray 8 there is provided connection means 22 for connecting the distal element 2 to the bearing element 4. The connection means 22 comprises a circumferential groove 24 which is disposed around the distal element 2. Proximal to the groove 24 is a circumferential lip 28 which projects laterally from the distal element 2. The lip 28 has a side wall 30 extending around its periphery.

With reference to Figure 5, the side wall 30 of the lip 28 is interrupted by four equidistantly spaced recesses 50a-d.

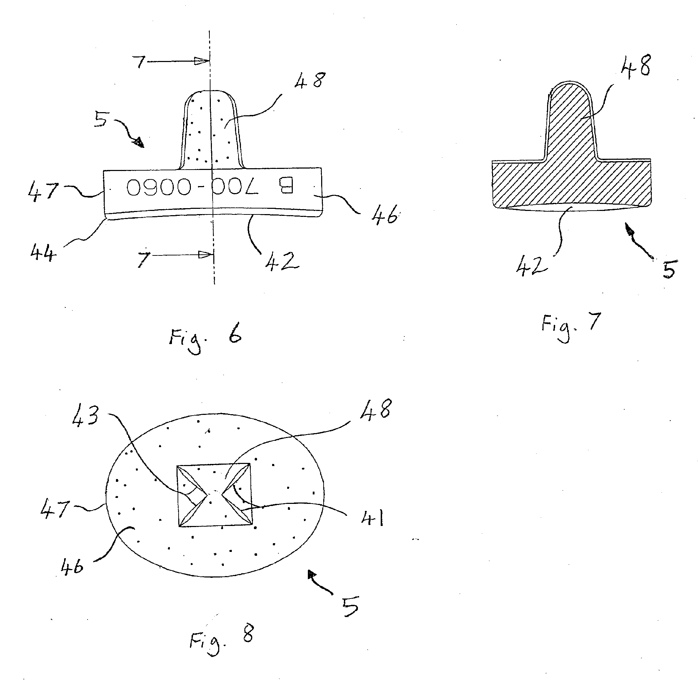

A proximal element 5 is illustrated in Figures 6 to 8. It comprises an oval shaped tray 46 having a concave bearing surface 42 situated on its distal face. A vertebral stem 48 extends perpendicularly away from a centre of the proximal face of the tray 46. The tray 46 includes a side wall 47 which extends around the circumference of the tray 46 and has a bevelled distal edge 44.

In use of the prosthesis 1, a surgeon removes damaged tissue, such as a cervical disc and drills anchorage holes in adjacent faces of the vertebrae which abut the damaged disc. The vertebral stem 6 of the distal element 2 is fixed in the anchorage hole in one of the vertebrae, and the vertebral stem 48 of the proximal element 5 is fixed in the anchorage hole in the other vertebra. Bone cement may be used to secure the vertebral stems 6, 48 in position.

The oval trays 8, 46 of the distal and proximal elements 2, 5 are aligned such that their greatest width lies along a medio-lateral axis of the human body, and their least width lies in the saggital plane. Furthermore, the concave surface 42 of the proximal element 5 engages the convex surface 32 of the bearing element 18 in a load bearing manner. The concave bearing surface 42 is thus superior to the convex bearing surface 32, in vivo.

The prosthesis 1 allows the vertebrae to which it is connected to rotate relative to one another to a certain degree, around medial-lateral and anterior-posterior axes. The prosthesis also allows relative rotation of the vertebrae about a longitudinal axis of the spine. Load bearing stability of the prosthesis is provided by the surrounding muscle tissue, but is enhanced by the self centring action of the concave bearing surface 42 and convex bearing surface 32.

## Claims

1. A prosthesis for replacing a part of a human or animal spine, the prosthesis comprising a first element which is adapted to be connected to a first vertebra and a second element which is adapted to be connected to a second vertebra of the spine, the first element being capable of articulation relative to the second element in vivo.

2. A prosthesis as claimed in claim 1, in which a bearing element is interposed between the first element and the second element.

3. A prosthesis as claimed in claim 2, in which the bearing element is attached to the first element.

4. A prosthesis as claimed in claim 2 or 3, in which the second element has a bearing surface which in an implanted condition of the prosthesis engages a cooperating wear surface of the bearing element.

5. A prosthesis as claimed in any one of claims 2 to 4, in which the wear surface of the bearing element and the bearing surface of the second element are shaped to reduce the likelihood of dislocation.

6. A prosthesis as claimed in claim 5, in which the wear surface of the bearing element is convex and the bearing surface of the second element is concave.

7. A prosthesis as claimed in any one of claims 2 to 5, in which the wear surface of the bearing element is concave and the bearing surface of he second element is convex.

8. A prosthesis as claimed in any one of claims 2 to 7, wherein the bearing element is made from ultrahigh molecular weight polyethylene.

9. A prosthesis as claimed in any one of claims 2 to 8, in which the bearing element is provided with cooperating formations which engage with cooperating formations on the first element.

10. A prosthesis as claimed in any one of claims 2 to 9, in which the bearing element is moulded onto the first element.

11. A prosthesis as claimed in claim 10, in which the bearing element is compression moulded onto the first element.

12. A prosthesis as claimed in any one of claims 2 to 11, in which the bearing element is fixed permanently to the first element.

13. A prosthesis as claimed in any one of the preceding claims, in which the first element and/or the second element are made from stainless steel or titanium.

14. A kit of parts comprising a prosthesis as claimed in any one of claims 2 to 13, the kit comprising a plurality of first elements, each first element having a bearing element of different shape.

15. A kit of parts as claimed in claim 14, in which a wear surface of the bearing element of one of the said first elements is offset from a central axis of that first element.

16. A prosthesis substantially as described herein with reference to, and as shown in, the accompanying drawings.
